Europäisches Patentamt

European Patent Office

Office européen des brevets

(11) Veröffentlichungsnummer: **0 512 350 A2**

## EUROPÄISCHE PATENTANMELDUNG

(21) Anmeldenummer: **92107132.0**

(22) Anmeldetag: **27.04.92**

(51) Int. Cl.⁵: **C07D 453/02**, A61K 31/435

(30) Priorität: **10.05.91 DE 4115215**

(43) Veröffentlichungstag der Anmeldung:
**11.11.92 Patentblatt 92/46**

(84) Benannte Vertragsstaaten:
**AT BE CH DE DK ES FR GB GR IT LI LU NL PT
SE**

(71) Anmelder: **MERCK PATENT GESELLSCHAFT
MIT BESCHRÄNKTER HAFTUNG
Frankfurter Strasse 250 Postfach 4119
W-6100 Darmstadt(DE)**

(72) Erfinder: **Böttcher, Henning, Dr.
Soderstrasse 95
W-6100 Darmstadt(DE)**
Erfinder: **Seyfried, Christoph, Dr.
Mathildenstrasse 6
W-6104 Jugenheim(DE)**
Erfinder: **Bartoszyk, Gerd
Heinrich-Fulda-Weg 22
W-6100 Darmstadt(DE)**
Erfinder: **Minck, Klaus-Otto, Dr.
Büchestrasse 8
W-6105 Ober-Ramstadt(DE)**

(54) **Indolderivate.**

(57) 3-(Indol-2-carboxamido)-chinuclidine der Formel I

Ind-CO-NHR      I

worin

Ind     eine unsubstituierte oder eine ein- bis dreifach durch Alkyl, Alkoxy, Alkoxycarbonyl und/oder Alkylthio mit jeweils 1-4 C-Atomen, 7-11 C-Aralkyloxy, 1-5 C-Acyloxy, 6-10 C-Aroyloxy, 6-10 C-Aryloxy, Trifluormethyl, Cyan, Fluor, Chlor, Hydroxy, 1-4 C-Alkylsulfonyloxy, 6-10 C-Arylsulfonyloxy, Carboxy und/oder Methylendioxy substituierte 2-Indolylgruppe und

R       3-Chinuclidinyl
        bedeuten
sowie deren physiologisch unbedenklichen Salze, die selektive serotonin-antagonistische Wirkung zeigen und als Psychopharmaka verwendet werden können.

Gegenstand der Erfindung sind neue 3-(Indol-2-carboxamido)-chinuclidin-Derivate der Formel I

Ind-CO-NHR    I

worin

Ind    eine unsubstituierte oder eine ein- bis dreifach durch Alkyl, Alkoxy, Alkoxycarbonyl und/oder Alkylthio mit jeweils 1-4 C-Atomen, 7-11 C-Aralkyloxy, 1-5 C-Acyloxy, 6-10 C-Aroyloxy, 6-10 C-Aryloxy, Trifluormethyl, Cyan, Fluor, Chlor, Hydroxy, 1-4 C-Alkylsulfonyloxy, 6-10 C-Arylsulfonyloxy, Carboxy und/oder Methylendioxy substituierte 2-Indolylgruppe und

R    3-Chinuclidinyl
bedeuten
sowie deren Salze.

Der Erfindung lag die Aufgabe zugrunde, neue Verbindungen aufzufinden, die zur Herstellung von Arzneimitteln verwendet werden können.

Es wurde gefunden, daß die genannten Substanzen bei guter Verträglichkeit wertvolle pharmakologische Eigenschaften besitzen. Diese Wirkungen erlauben es, diese Substanzen zur Behandlung von Erkrankungen, die durch einen Überschuß an zirkulierendem Serotonin oder durch eine serotonerge Überfunktion charakterisiert sind, einzusetzen. Dazu gehören insbesondere die Behandlung von Psychosen, von Nausea und Erbrechen (wie sie z.B. bei der chemo- oder radiotherapeutischen Behandlung von Krebserkrankungen auftreten), von Dementia oder anderen kognitiven Erkrankungen, von Migräne und von Suchterkrankungen. Dazu gehören weiterhin die Anwendung als Anxiolytikum, als Antiaggressivum, als Antidepressivum und als Analgetikum. Im einzelnen antagonisieren die Verbindungen die Wirkung von Serotonin an 5-HT$_3$-Rezeptoren, wie z.B. den durch Serotonin hervorgerufenen von-Bezold-Jarisch-Reflex (Methodik siehe J.Pharm.Pharmacol. 40 (1980), 301-302 und Nature 316 (1985), 126-131). Außerdem verdrängen die neuen Verbindungen die als selektiver 5-HT$_3$-Ligand bekannte Substanz $^3$H-GR65630 von homogenisiertem Gewebe aus dem endorhinalen Cortex der Ratte (siehe Europ.J.Pharmacol. 159 (1989), 157-164).

Die Verbindungen I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Ähnliche Verbindungen sind in J.Org.Chem. 40, 2525-2529 (1975), in J.Org. Chem. 38, 3004-3011 (1973) und in J.Org.Chem. 33, 487-490 (1968) beschrieben. In allen diesen Fällen sind jedoch keine pharmakologischen Wirkungen angegeben.

Im Rest Ind bedeuten die Substituenten 1-4-C-Alkyl vorzugsweise Methyl, ferner auch Ethyl, n-Propyl, Isopropyl, n-Butyl, Isobutyl, sek.-Butyl oder tert.-Butyl. 1-4-C-Alkoxy ist vorzugsweise Methoxy, ferner auch Ethoxy, n-Propoxy, Isopropoxy, n-Butoxy, Isobutoxy, sek.-Butoxy oder tert.-Butoxy, 1-5-C-Acyloxy vorzugsweise Formyloxy oder Acetyloxy, Propanoyloxy, n-Butanoyloxy, Isobutanoyloxy oder Pivaloyloxy. 1-4-C-Alkylsulfonyloxy ist vorzugsweise Methansulfonyloxy. 6-10-C-Aryloxy ist bevorzugt Phenyloxy, 7-11-C-Aralkyloxy ist bevorzugt Benzyloxy, 6-10-C-Aroyloxy ist bevorzugt Benzoyloxy.

Bevorzugt bedeutet der Rest Ind eine unsubstituierte oder einfach oder zweifach substituierte 2-Indolylgruppe. Falls Ind eine substituierte 2-Indolylgruppe bedeutet, so ist sie vorzugsweise in 4-, 5- und/oder 6-Stellung substituiert und/oder in 1-Stellung alkyliert. Im einzelnen bedeutet Ind vorzugsweise 2-Indolyl, 5-Methoxy-2-indolyl, 1- oder 5-Methyl-2-indolyl, oder 4-Chlor- oder 4-Fluor-2-indolyl. Der Rest R ist 3-Chinuclidinyl.

Gegenstand der Erfindung ist ferner ein Verfahren zur Herstellung von Verbindungen der Formel I sowie von ihren Salzen, dadurch gekennzeichnet, daß man Verbindungen der Formel II,

Ind-CO-X    II

wobei Ind die angegebene Bedeutung hat,
und
X Chlor, Brom, Acyloxy, vorzugsweise Pivaloyloxy, ferner Acetyloxy, Propionyloxy oder Butanoyloxy, 1-6 C-Alkoxy, vorzugsweise Methoxy oder Ethoxy, 7-11 C-Aralkyloxy, vorzugsweise Benzyloxy oder 6-10 C-Aroyloxy, vorzugsweise Benzoyloxy, oder Hydroxy bedeutet,
mit 3-Aminochinuclidin umsetzt, und/oder eine Verbindung, die sonst der Formel I entspricht, aber anstelle eines oder mehrerer H-Atome eine abspaltbare Schutzgruppe enthält, durch Abspaltung dieser Schutzgruppe in eine Verbindung der Formel I überführt und/oder in einer Verbindung der Formel I einen Rest Ind in einen anderen Rest Ind umwandelt und/oder eine Base der Formel I durch Behandeln mit einer Säure in eines ihrer Salze umwandelt und/oder aus einem Salz einer Base der Formel I diese mittels einer starken Base freisetzt.

Die Herstellung von Verbindungen der Formel I erfolgt im übrigen nach an sich bekannten Methoden, wie sie in der Literatur (z.B. J. March, Advanced Organic Chemistry, 3rd edition, John Wiley & Sons, New York; F.M. Finn et al. The Proteins 3rd ed. Vol. II Chap. 2 oder Houben-Weyl, Methoden der Organischen Chemie, Georg-Thieme-Verlag, Stuttgart) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu einer Verbindung der Formel I umsetzt.

Die Herstellung einer Verbindung der Formel I erfolgt nach an sich bekannten Methoden, wie sie im allgemeinen zur Herstellung von Amiden oder in der Peptidchemie verwendet werden, vorzugsweise, indem man eine Verbindung der Formel II mit 3-Aminochinuclidin oder einem seiner Salze in einem geeigneten Lösungsmittel, wie z.B. Tetrahydrofuran (THF), Dimethylformamid (DMF) 1,4-Dioxan, Alkoholen wie z.B. Methanol oder Ethanol, weiteren Ethern wie z.B. Diethylether, Dichlormethan oder aber auch Mischungen der genannten Lösungsmittel, bei Temperaturen zwischen 10° und dem jeweiligen Siedepunkt des verwendeten Lösungsmittels, gegebenenfalls unter Zusatz eines Aktivators oder eines Katalysators, wie z.B. 4-(Dimethylamino)-pyridin oder N,N'-Dicyclohexylcarbodiimid, aber auch Pivalinsäurechlorid, über eine Zeitdauer von 0,5 bis 72 Stunden umsetzt.

Die Verbindungen der Formel I enthalten zumindestens ein asymmetrisches Kohlenstoffatom. Sie können daher als Racemate, falls mehrere asymmetrische Kohlenstoffatome vorhanden sind, auch als Gemische mehrerer Racemate sowie in verschiedenen optisch-aktiven Formen vorliegen. Weisen die Verbindungen zwei oder mehr Asymmetriezentren auf, dann fallen sie bei der Synthese im allgemeinen als Gemisch von Racematen an, aus denen man die einzelnen Racemate, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet. Als Trennmittel eignen sich z.B. optisch aktive Säuren, wie die D- und L-Formen von Weinsäure, Dibenzoylweinsäure, Diacetylweinsäure, Camphersulfonsäuren, Mandelsäure, Äpfelsäure oder Milchsäure. Die verschiedenen Formen der Diastereomeren können in an sich bekannter Weise, z.B. durch fraktionierte Kristallisation, getrennt, und die optisch aktiven Verbindungen der Formel I können in an sich bekannter Weise aus den Diastereomeren in Freiheit gesetzt werden.

Die Verbindungen der Formel II sind zum Teil bekannt; die nicht bekannten Verbindungen können leicht analog zu den bekannten Verbindungen hergestellt werden. So können z.B. N- oder O-acylierte Verbindungen aus den nicht-acylierten Vorstufen durch Umsetzung mit Säureanhydriden, z.B. Acetanhydrid, in basischen organischen Lösungsmitteln, z.B. Pyridin, hergestellt werden.

Ebenso können die an sich bekannten Methoden zur Derivatisierung von Carbonsäuren zur Herstellung von Verbindungen der Formel II verwendet werden.

Man kann weiterhin aus Verbindungen, die sonst der Formel I entsprechen, aber anstelle eines oder mehrerer H-Atome eine abspaltbare Schutzgruppe, insbesondere eine hydrogenolytisch abspaltbare Schutzgruppe, wie Benzyloxy, enthalten, diese mittels katalytischer Hydrierung abspalten, wobei man Verbindungen der Formel I erhält. Weiterhin können im besonderen Verbindungen, die sonst der Formel I entsprechen, aber anstelle eines oder mehrerer H-Atome eine solvolytisch abspaltbare Schutzgruppe, wie Acyl (z.B. Acetyl) oder Sulfonyl (z.B. Methansulfonyl oder Toluolsulfonyl), enthalten, zu Verbindungen der Formel I solvolysiert, insbesondere hydrolysiert werden.

Die Ausgangsstoffe für die Solvolyse sind beispielsweise erhältlich durch Reaktionen von 1-Z-Ind-COX mit 3-Aminochinuclidin oder einem seiner Salze, wobei Ind die angegebene Bedeutung besitzt und Z eine solvolytisch abspaltbare Gruppe ist. So können insbesondere Verbindungen der Formel I, wobei der Rest Ind in der 1-Stellung des Indols eine Acylgruppe, vorzugsweise eine Alkanoyl-, Alkylsulfonyl- oder Arylsulfonylgruppe mit jeweils bis zu 10 C-Atomen, wie Methan-, Benzol- oder p-Toluolsulfonyl, enthält, zu den entsprechenden in 1-Stellung unsubstituierten Verbindungen hydrolysiert werden, z.B. in saurem, besser neutralem oder alkalischem Medium bei Temperaturen zwischen 0 und 200 °C. Als Basen verwendet man zweckmäßig Natrium-, Kalium- oder Calciumhydroxid, Natrium- oder Kaliumcarbonat oder Ammoniak, unter Bedingungen, bei denen die Säureamidbindung nicht gespalten wird. Als Lösungsmittel wählt man vorzugsweise Wasser, niedere Alkohole wie Methanol oder Ethanol, Ether wie THF oder Dioxan, Sulfone wie Tetramethylensulfon oder deren Gemische, besonders die Wasser enthaltenden Gemische. Eine Hydrolyse kann bereits beim Behandeln mit Wasser allein erfolgen, insbesondere in der Siedehitze.

Man kann in einer Verbindung der Formel I einen Rest Ind in einen anderen Rest Ind umwandeln, sofern die gewählten Reaktionsbedingungen die Säureamidbindung nicht zerstören, indem man z.B. eine Ethergruppe spaltet, wobei das entsprechende Hydroxyderivat entsteht, und/oder eine Carboxylgruppe verestert und/oder eine Estergruppe verseift und/oder eine Carboxylgruppe durch Decarboxylierung abspaltet. So kann man die Ether spalten durch Behandeln mit Dimethylsulfid-Bortribromid-Komplex, z.B. in Toluol, Ethern wie THF oder Dimethylsulfoxid, oder durch Verschmelzen mit Pyridin- oder Anilinhydrohalogeniden, vorzugsweise Pyridinhydrochlorid, bei etwa 150-250 °C, oder durch Behandeln mit Diisobutylaluminiumhydrid in Toluol bei etwa 0-110 °C, oder durch katalytische Hydrierung, z.B. in Gegenwart von Palladium-Kohle in einem der oben genannten inerten Lösungsmitteln, z.B. Methanol, bei z.B. 0 bis 50 °C und bei z.B. 1 bis 10 bar. Die genannten Veresterungen erfolgen z.B. durch die Behandlung einer Lösung der Carbonsäure mit einem Alkohol unter Zusatz von $SOCl_2$ oder eines wasserabspaltenden Mittels, wobei vorzugsweise ein Überschuß des Alkohols als Lösungsmittel dient. Die Hydrolyse von Carbonsäureestern erfolgt z.B. mittels saurer oder basischer Katalyse in einer wäßrigen Lösung, die zusätzlich ein inertes mit Wasser mischbares organisches Lösungsmittel, wie Dioxan, enthalten kann. Decarboxylierungen werden zweckmäßigerweise in alkalischem Medium, z.B. in N,N-Dimethylanilin bei Temperaturen zwischen 40 und 190 °C, vorzugsweise zwischen 160 und 190 °C, ausgeführt.

Eine erhaltene Base der Formel I kann mit einer Säure in das zugehörige Säureadditionssalz übergeführt werden. Für diese Umsetzung eignen sich bevorzugt Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- und Ethansulfonsäure, Benzolsulfonsäure, p-Toluolsulfonsäure, Naphthalin-mono- und -disulfonsäuren, Laurylschwefelsäure. Säureadditionssalze, die nicht physiologisch unbedenklich sind (z.B. Pikrate), können sich zur Isolierung und Aufreinigung der Basen der Formel I eignen.

Eine Base der Formel I kann, falls erwünscht, aus einem ihrer Salze mit starken Basen wie Natrium- oder Kaliumhydroxid, Natrium- oder Kaliumcarbonat in Freiheit gesetzt werden.

Gegenstand der Erfindung ist ferner die Verwendung von Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoffe(n) in eine geeignete Darreichungsform gebracht werden.

Gegenstand der Erfindung sind ferner Mittel, insbesondere pharmazeutische Zubereitungen, enthaltend eine oder mehrere Verbindungen der Formel I und/oder ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlenhydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wäßrige Lösungen, ferner Suspensionen, Emulsionen oder Implantate, für die topische Anwendungen Salben, Cremes, Pflaster oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht auch einen oder mehrere weitere Wirkstoffe enthalten, z.B. ein oder mehrere Vitamine.

Verbindungen der Formel I und ihre physiologisch unbedenklichen Salze können bei der therapeutischen Behandlung des menschlichen oder tierischen Körpers und bei der Bekämpfung von Krankheiten verwendet werden, insbesondere von Krankheiten, die mit einem Überschuß an zirkulierendem Serotonin oder durch eine serotonerge Überfunktion gekennzeichnet sind. Zu diesen Krankheiten gehören insbesondere Psychosen, Nausea und Erbrechen, wie sie als Begleitsymptome bei der chemo- und radiotherapeutischen Behandlung von Tumoren entstehen, Dementia und andere kognitive Erkrankungen, Migräne und Suchterkrankungen. Weiterhin gehören anxiolytische, analgetische, anti-depressive und anti-aggressive Wirkung in diesen Anwendungsbereich.

Dabei werden die erfindungsgemäßen Substanzen in der Regel in Analogie zu bekannten, im Handel befindlichen Präparaten (Thioridazin, Haloperidol) verabreicht, vorzugsweise in Dosierungen zwischen etwa 0,2 und 1000 mg, insbesondere zwischen 0,2 und 100 mg pro Dosierungseinheit. Die tägliche Dosierung liegt vorzugsweise zwischen etwa 0.003 und 20 mg/kg Körpergewicht. Die spezielle Dosis für jeden einzelnen Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Anwendung ist bevorzugt.

Beispiele

In den nachstehenden Beispielen bedeutet "übliche Aufarbeitung": Man gibt, falls erforderlich, Wasser hinzu, extrahiert mit Ethylacetat oder Ether, trennt ab, trocknet die organische Phase über Natriumsulfat, filtriert, dampft ein und reinigt durch Chromatographie an Kieselgel und/oder durch Kristallisation. Temperaturen sind in Grad Celsius angegeben.

Die Festpunkte beziehen sich, sofern nichts anderes angegeben ist, auf die freien Basen.

**Beispiel 1**

Zu einer Lösung von 2,4 g Indol-2-carbonsäure in 70 ml THF fügt man die äquimolare Menge Pivalinsäurechlorid und 2 ml Pyridin hinzu. Anschließend werden bei Raumtemperatur 3,1 g 3-Aminochinuclidin in 10 ml THF zugegeben, und es wird 3 Stunden gekocht. Nach üblicher Aufarbeitung erhält man Indol-2-carbonsäure-N-(chinuclidin-3-yl)-amid, F. 178-80°.

Analog erhält man durch Umsetzung von 3-Aminochinuclidin

mit 5-Methylindol-2-carbonsäure 5-Methylindol-2-carbonsäure-N-(chinuclidin-3-yl)-amid, F. 163-165°;

mit 6-Methoxyindol-2-carbonsäure 6-Methoxyindol-2-carbonsäure-N-(chinuclidin-3-yl)-amid,

**mit 4-Benzyloxyindol-2-carbonsäure 4-Benzyloxyindol-2-carbonsäure-N-(chinuclidin-3-yl)-amid,**

mit 5-Methoxyindol-2-carbonsäure 5-Methoxyindol-2-carbonsäure-N-(chinuclidin-3-yl)-amid, F. 250-52°,

mit 4-Fluorindol-2-carbonsäure 4-Fluorindol-2-carbonsäure-N-(chinuclidin-3-yl)-amid, **F. 255-257°,**

mit 5-Fluorindol-2-carbonsäure 5-Fluorindol-2-carbonsäure-N-(chinuclidin-3-yl)-amid, **F. 316-318°,**

mit 1-Ethylindol-2-carbonsäure 1-Ethylindol-2-carbonsäure-N-(chinuclidin-3-yl)-amid,

mit 5-Ethoxyindol-2-carbonsäure 5-Ethoxyindol-2-carbonsäure-N-(chinuclidin-3-yl)-amid,

mit 6-Ethoxyindol-2-carbonsäure 6-Ethoxyindol-2-carbonsäure-N-(chinuclidin-3-yl)-amid,

mit 7-Ethoxyindol-2-carbonsäure 7-Ethoxyindol-2-carbonsäure-N-(chinuclidin-3-yl)-amid,

mit 5-Cyanindol-2-carbonsäure 5-Cyanindol-2-carbonsäure-N-(chinuclidin-3-yl)-amid,

mit 6-Cyanindol-2-carbonsäure 6-Cyanindol-2-carbonsäure-N-(chinuclidin-3-yl)-amid,

mit 7-Cyanindol-2-carbonsäure 7-Cyanindol-2-carbonsäure-N-(chinuclidin-3yl)-amid,

mit 4-Chlorindol-2-carbonsäure 4-Chlorindol-2-carbonsäure-N-(chinuclidin-3-yl)-amid, F. 240-42°,

mit 5-Chlorindol-2-carbonsäure 4-Chlorindol-2-carbonsäure-N-(chinuclidin-3-yl)-amid,

mit 6-Chlorindol-2-carbonsäure 6-Chlorindol-2-carbonsäure-N-(chinuclidin-3-yl)-amid,

mit 5,6-Dimethoxyindol-2-carbonsäure 5,6-Dimethoxyindol-2-carbonsäure-N-(chinuclidin-3-yl)-amid,

mit 5,6-Methylendioxyindol-2-carbonsäure 5,6-Methylendioxyindol-2-carbonsäure-N-(chinuclidin-3-yl)-amid,

mit 4,5,6-Trimethoxyindol-2-carbonsäure 4,5,6-Trimethoxyindol-2-carbonsäure-N-(chinuclidin-3-yl)-amid,

mit 5-Trifluormethylindol-2-carbonsäure 5-Trifluormethylindol-2-carbonsäure-N-(chinuclidin-3-yl)-amid,

mit 6-Trifluormethylindol-2-carbonsäure 6-Trifluormethylindol-2-carbonsäure-N-(chinuclidin-3-yl)-amid,

**mit 5-Benzyloxyindol-2-carbonsäure 5-Benzyloxyindol-2-carbonsäure-N-(chinuclidin-3-yl)-amid,** F. 283-284°,

mit 5-Benzylindol-2-carbonsäure 5-Benzylindol-2-carbonsäure-N-(chinuclidin-3-yl)-amid,

mit 6-Benzylindol-2-carbonsäure 6-Benzylindol-2-carbonsäure-N-(chinuclicin-3-yl)-amid,

mit 6-Benzyl-1-methyl-indol-2-carbonsäure 6-Benzyl-1-methyl-indol-2-carbonsäure-N-(chinuclidin-3-yl)-amid,

mit 6-Chlor-1-methyl-indol-2-carbonsäure 6-Chlor-1-methyl-indol-2-carbonsäure-N-(chinuclidin-3-yl)-amid,

mit 6-Cyan-1-methyl-indol-2-carbonsäure 6-Cyan-1-methyl-indol-2-carbonsäure-N-(chinuclidin-3-yl)-amid,

mit 1-Methylindol-2-carbonsäure 1-Methylindol-2-carbonsäure-N-(chinuclidin-3yl)-amid, F. 182-84°,

mit 7-Benzylindol-2-carbonsäure 7-Benzylindol-2-carbonsäure-N-(chinuclidin-3-yl)-amid,

mit 6-Ethoxy-1-methyl-indol-2-carbonsäure 6-Ethoxy-1-methyl-indol-2-carbonsäure-N-(chinuclidin-3-yl)-amid,

**mit 6-Benzyloxyindol-2-carbonsäure 6-Benzyloxyindol-2-carbonsäure-N-(chinuclidin-3-yl)-amid,**

**mit 7-Benzyloxyindol-2-carbonsäure 7-Benzyloxyindol-2-carbonsäure-N-(chinuclidin-3-yl)-amid;**
**F. 268-269°,**

mit 5-Cyan-1-methyl-indol-2-carbonsäure 5-Cyan-1-methyl-indol-2-carbonsäure-N-(chinuclidin-3-yl)-amid,

mit 6-Propoxyindol-2-carbonsäure 6-Propoxyindol-2-carbonsäure-N-(chinuclidin-3-yl)-amid,

mit 7-Fluorindol-2-carbonsäure 7-Fluorindol-2-carbonsäure-N-(chinuclidin-3-yl)-amid,

mit 4-Trifluormethylindol-2-carbonsäure 4-Trifluormethylindol-2-carbonsäure-N-(chinuclidin-3-yl)-amid,

mit 5-Ethylindol-2-carbonsäure 5-Ethylindol-2-carbonsäure-N-(chinuclidin-3-yl)-amid,

mit 6-tert.-Butylindol-2-carbonsäure 6-tert.-Butylindol-2-carbonsäure-N-(chinuclicin-3-yl)-amid und

mit 6-Acetoxy-1-methyl-indol-2-carbonsäure 6-Acetoxy-1-methyl-indol-2-carbonsäure-N-(chinuclidin-3-yl)-amid.

### Beispiel 2

300 mg 5-Hydroxyindol-2-carbonsäure-N-(chinuclidin-3-yl)-amid werden in 20 ml Pyridin gelöst und mit 8 ml Acetanhydrid unter Kühlung versetzt. Danach wird 1 Stunde bei Raumtemperatur gerührt und wie üblich aufgearbeitet. Man erhält 5-Acetoxyindol-2-carbonsäure-N-(chinuclidin-3-yl)-amid.

Analog erhält man durch Umsetzung der 4-, 5- oder 6-Hydroxyindol-2-carbonsäuren mit den entsprechend Carbonsäureanhydriden
5-Pivaloyloxyindol-2-carbonsäure-N-(chinuclidin-3-yl)-amid,
6-Pivaloyloxyindol-2-carbonsäure-N-(chinuclidin-3-yl)-amid,
6-Butanoyloxyindol-2-carbonsäure-N-(chinuclidin-3-yl)-amid,
5-Butanoyloxyindol-2-carbonsäure-N-(chinuclidin-3-yl)-amid,
4-Propanoyloxyindol-2-carbonsäure-N-(chinuclidin-3-yl)-amid,
5-Propanoyloxyindol-2-carbonsäure-N-(chinuclidin-3-yl)-amid,
6-Propanoyloxyindol-2-carbonsäure-N-(chinuclidin-3-yl)-amid,
4-Methansulfonyloxyindol-2-carbonsäure-N-(chinuclidin-3-yl)-amid, amid,
5-Methansulfonyloxyindol-2-carbonsäure-N-(chinuclidin-3-yl)-amid, amid,
6-Methansulfonyloxyindol-2-carbonsäure-N-(chinuclidin-3-yl)-amid, amid,
5-Propanoyloxy-1-methyl-indol-2-carbonsäure-N-(chinuclidin-3-yl)-amid,
4-Methansulfonyloxy-1-methyl-2-indol-2-carbonsäure-N-(chinuclidin-3yl)-amid,
5-Methansulfonyloxy-1-methyl-indol-2-carbonsäure-N-(chinuclidin-3-yl)-amid und
5-Acetoxy-1-methyl-indol-2-carbonsäure-N-(chinuclidin-3-yl)-amid.

### Beispiel 3

**0,5 g 7-Benzyloxyindol-2-carbonsäure-N-(chinuclidin-3-yl)-amid (F. 268-269°) werden in 40 ml Methanol gelöst und nach Zugabe von 0,2 g Palladium-Kohle(5%) bei Raumtemperatur über eine Zeitdauer von 1 Stunde hydriert.Anschließend wird die** Hydrierlösung eingeengt und wie üblich aufgearbeitet. Man erhält 7-Hydroxyindol-2-carbonsäure-N-(chinuclidin-3-yl)-amid.

Analog erhält man durch Hydrierung
von 5-Benzyloxyindol-2-carbonsäure-N-(chinuclidin-3-yl)-amid
**(F. 283-284°),**
**5-Hydroxyindol-2-carbonsäure-N-(chinuclidin-3-yl)amid,**
von 6-Benzyloxyindol-2-carbonsäure-N-(chinuclidin-3-yl)-amid 6-Hyroxyindol-2-carbonsäure-N-(chinuclidin-3-yl)-amid und
von 4-Benzyloxyindol-2-carbonsäure-N-(chinuclidin-3-yl)-amid 4-Hydroxyindol-2-carbonsäure-N-(chinuclidin-3-yl)-amid.

### Beispiel 4

2,7 g 5-Methylindol-2-carbonsäure werden unter Zugabe von 3,0 g Dicyclohexylcarbodiimid (DCC) in 100 ml THF bei Raumtemperatur gelöst und mit 3,2 g 3-Aminochinuclidin suspendiert in 20 ml THF versetzt. Anschließend wird 6 Stunden bei 40° gerührt und wie üblich aufgearbeitet. Man erhält 5-Methylindol-2-carbonsäure-N-(chinuclidin-3-yl)-amid.

Die nachstehenden Beispiele betreffen pharmazeutische Zubereitungen, die Substanzen der Formel I oder eines ihrer Säureadditionssalze enthalten:

### Beispiel A: Tabletten

Ein Gemisch von 1 kg 4-Chlorindol-2-carbonsäure-N-(chinuclidin-3-yl)-amid, 4 kg Lactose, 1,2 kg Kartoffelstärke, 0,2 kg Talk und 0,1 kg Magnesiumstearat wird in üblicher Weise zu Tabletten verpreßt, so daß jede Tablette 10 mg Wirkstoff enthält.

**Beispiel B: Dragees**

Analog Beispiel A werden Tabletten gepreßt, die anschließend in üblicher Weise mit einem Überzug aus Saccharose, Kartoffelstärke, Talk, Tragant und Farbstoff überzogen werden.

**Beispiel C: Kapseln**

2 kg 5-Methoxyindol-2-carbonsäure-N-(chinuclidin-3-yl)-amid werden in üblicher Weise in Hartgelatinekapseln gefüllt, so daß jede Kapsel 20 mg Wirkstoff enthält.

**Beispiel D: Ampullen**

Eine Lösung von 1 kg Indol-2-carbonsäure-N-(chinuclidin-3-yl)-amid in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die eine andere Verbindung der Formel I und/oder ein oder mehrere physiologisch unbedenkliche Säureadditionssalze einer Verbindung der Formel I enthalten.

**Patentansprüche**

1. 3-(Indol-2-carboxamido)-chinuclidine der Formel I

    Ind-CO-NHR    I

    worin

    Ind  eine unsubstituierte oder eine ein- bis dreifach durch Alkyl, Alkoxy, Alkoxycarbonyl und/oder Alkylthio mit jeweils 1-4 C-Atomen, 7-11 C-Aralkyloxy, 1-5 C-Acyloxy, 6-10 C-Aroyloxy, 6-10C-Aryloxy, Trifluormethyl, Cyan, Fluor, Chlor, Hydroxy, 1-4 C-Alkylsulfonyloxy, 6-10 C-Arylsulfonyloxy, Carboxy und/oder Methylendioxy substituierte 2-Indolylgruppe

    und

    R  3-Chinuclidinyl

    bedeuten

sowie deren Salze.

2.
    a) 4-Chlor-indol-2-carbonsäure-N-(chinuclidin-3-yl)-amid
    b) 5-Methoxy-indol-2-carbonsäure-N-(chinuclidin-3-yl)-amid
    c) Indol-2-carbonsäure-N-(chinuclidin-3-yl)-amid
    d) 1-Methyl-indol-2-carbonsäure-N-(chinuclidin-3-yl)-amid
    e) 5-Methyl-indol-2-carbonsäure-N-(chinuclidin-3-yl)-amid
    f) 4-Fluor-indol-2-carbonsäure-N-(chinuclidin-3-yl)-amid

3. Verfahren zur Herstellung von Verbindungen der Formel I gemäß Anspruch 1 sowie von deren Salzen, dadurch gekennzeichnet, daß man Verbindungen der Formel II

    Ind-CO-X    II

    wobei der Rest Ind die in Anspruch 1 angegebene Bedeutung hat und X Cl, Br, Acyloxy, Alkoxy mit jeweils 1-6 C-Atomen, Aralkyloxy mit 7-11 C-Atomen, Aroyloxy, mit jeweils 6-10 C-Atomen oder Hydroxy bedeuten, mit 3-Amino-chinuclidin umsetzt und/oder eine Verbindung, die sonst der Formel I entspricht, aber anstelle eines oder mehrerer H-Atome eine abspaltbare Schutzgruppe enthält, durch Abspaltung dieser Schutzgruppe in eine Verbindung der Formel I überführt und/oder in einer Verbindung der Formel I einen Rest Ind in einen anderen Rest Ind und/oder eine Base der Formel I durch Behandeln mit einer Säure in eines ihrer Salze umwandelt.

4. Verfahren zur Herstellung einer pharmazeutischen Zubereitung, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Darreichungsform bringt.

5. Pharmazeutische Zubereitung, gekennzeichnet durch einen Gehalt an mindestens einer Verbindung der Formel I gemäß Anspruch 1 und/oder einem ihrer physiologisch unbedenklichen Salze.

6. Verwendung einer Verbindung der Formel I gemäß Anspruch 1 und/oder eines ihrer physiologisch unbedenklichen Salze bei der Bekämpfung von Krankheiten.

**7.** Verwendung einer Verbindung der Formel I gemäß Anspruch 1 und/oder eines ihrer physiologisch unbedenklichen Salze zur Herstellung von Arzneimitteln.